# EUROPÄISCHE PATENTANMELDUNG

(11) **EP 1 470 803 A1**
(43) Veröffentlichungstag der Anmeldung: **27.10.2004**
(21) Anmeldenummer: 03405284.5
(22) Anmeldetag: 23.04.2003
(51) Int. Cl.: A61F 2/44, A61F 2/46

(54) **Vorrichtung für die Spondylodese**

(71) Anmelder: Sepitec Foundation, 9490 Vaduz (LI)
(72) Erfinder: Magerl, Friedrich, 9011 St. Gallen (CH); Stadler, Roger, 8001 Zürich (CH); Widmer, Christian, 9200 Gossau (CH)
(74) Vertreter: Groner, Manfred

(57) **Zusammenfassung**

Die Vorrichtung weist wenigstens ein Intervertebralimplantat (3) und wenigstens eine Platte (4,5) auf, die mit dem Intervertebralimplantat (3) und einem benachbarten Wirbel (21-23) zu verbinden ist. Das Intervertebralimplantat (3) ist mit wenigstens zwei im Abstand zueinander angeordneten Platten (4,5) verbunden. Beide Platten (4,5) bilden jeweils an einem Ende mit dem Intervertebralimplantat (3) ein fixierbares Gelenk. Vorzugsweise ist wenigstens eine der Platten (5,4) Z-förmig oder L-förmig ausgebildet. Die Vorrichtung ermöglicht eine hohe Anpassungsfähigkeit an die anatomischen Gegebenheiten der Wirbelsäule und bei kleinerer Teilezahl einen modularen Aufbau.

## Beschreibung

Die Erfindung betrifft eine Vorrichtung für die Spondylodese und insbesondere für die vordere interkorporelle Spondylodese an der Halswirbelsäule, mit wenigstens einem Intervertebralimplantat, und mit wenigstens einer Verbindungsplatte, die mit dem Intervertebralimplantat und einem benachbarten Wirbel zu verbinden ist.

Als Spondylodese wird die chirurgische Versteifung oder Verblockung eines Abschnittes der Wirbelsäule verstanden. Hierzu wird nach der klassischen Methode Knochen- oder Knochenersatzmaterial verwendet, welches zwischen die Wirbelkörper eingesetzt wird, sogenannte interkorporelle Spondylodese, oder über die hinteren Wirbelelemente angelagert wird, sogenannte dorsale Spondylodese. Hierdurch entsteht mit der Zeit eine knöcherne Brücke, welche die Wirbel verbindet, so dass sie sich nicht mehr gegeneinander bewegen können. Damit eine Verknöcherung stattfinden kann, ist es erforderlich, dass der betreffende Abschnitt der Wirbelsäule ruhiggestellt wird. Letzteres geschieht durch geeignete Implantate.

Mit konservativen Behandlungsmassnahmen nicht beeinflussbare Schmerzen, Rückenmark- oder Nervenwurzelkompressionen sowie Fehlstellungen sind Indikationen für Spondylodesen. Schmerzen können prinzipiell von allen krankhaft veränderten Strukturen der Wirbelsäule ausgehen. Für die Entwicklung von Rückenmarkoder Nervenwurzelkompressionen sind Verengungen des Wirbelkanals oder der Zwischenwirbellöcher verantwortlich. Mit einem chirurgischen Eingriff werden die pathologischen Veränderungen beseitigt und die Stabilität der Wirbelsäule durch die Spondylodese wieder hergestellt.

Weil bei der interkorporellen Spondylodese immer Bandscheiben ausgeräumt werden und dies die Stabilität der Wirbelsäule beeinträchtigt, ist diese immer wieder herzustellen. Dies kann beispielsweise mit druckfesten Knochenspänen geschehen, die man vom Patienten entnimmt, sogenannte autogene Knochenspäne, und zwischen den Wirbelkörpern einsetzt. Da die Belastbarkeit solcher Späne oft unsicher und ihre Verfügbarkeit begrenzt ist, und zudem die durch eine Spanentnahme verursachte Morbidität erheblich sein kann, benutzt man anstelle von autogenen Knochenspänen zunehmend aus körperfremden Materialien hergestellte Intervertebralimplantate, auch "Cages" genannt.

Ein Intervertebralimplantat funktioniert als druckaufnehmender Platzhalter, welcher die Spondylodese stabilisiert, die Einstellung der Wirbelkörper zueinander sichert und gewährleistet, dass sich zwischen den benachbarten Wirbelkörpern eine solide Knochenbrücke bilden kann. In die Intervertebralimplantate eingefülltes und/oder um sie herum angelagertes Knochen- oder Knochenersatzmaterial bilden eine Matrix für eine Knochenneubildung. Für den Ossifikationsprozess spielt die Stabilität der Spondylodese eine entscheidende Rolle. Innerhalb der Spondylodese stattfindende Bewegungen verzögern oder verhindern deren knöcherne Konsolidierung.

Im Stand der Technik sind zahlreiche Intervertebralimplantate bekannt geworden, die mit einer Platte mit Wirbeln verbunden werden.

So zeigt beispielsweise die US 6,235,059 B eine Vorrichtung mit einem Intervertebralimplantat, mit dem zwei Wirbel aneinander stabilisiert werden können. Am Intervertebralimplantat ist eine zweiarmige Platte befestigt, die starr oder schwenkbar mit dem Intervertebralimplantat verbunden ist. Jeder Arm besitzt eine Durchgangsbohrung, durch die eine Knochenschraube in den entsprechenden Wirbelkörper einschraubbar ist. Eine Anpassung an die jeweiligen anatomischen Gegebenheiten und insbesondere an die sagittale Krümmung ist hier sehr beschränkt. Das Intervertebralimplantat kann nur mit den benachbarten Wirbeln verbunden werden.

Die WO 00/24343 offenbart eine Vorrichtung mit einem Intervertebralimplantat, das am Umfang Windungen besitzt und ähnlich einer Schraube zwischen die Wirbel eingeschraubt werden kann. An einem vorderen Ende des Intervertebralimplantates ist eine flügelförmige zweiarmige Platte befestigt. Die Befestigung ist hier so ausgeführt, dass die Platte zur Anpassung an die anatomischen Gegebenheiten bewegbar ist. Die beiden Arme der Platte weisen jeweils ein Langloch für die Aufnahme einer Knochenschraube auf. Auch bei dieser Vorrichtung kann das Intervertebralimplantat nur mit den benachbarten Wirbeln verbunden werden. Die Anpassbarkeit an die anatomischen Gegebenheiten ist sehr beschränkt.

Die FR 2 727 005 offenbart eine Vorrichtung mit mehreren Intervertebralimplantaten, die mit einem gemeinsamen Band miteinander verbunden werden. Das Band weist Löcher zur Befestigung des Bandes und jeweils ein Intervertebralimplantat und Löcher zur Aufnahme einer Knochenschraube auf. Mit dieser Vorrichtung können zwar mehrere Intervertebralimplantate miteinander verbunden werden, die Anpassbarkeit an die anatomischen Gegebenheiten ist auch hier jedoch sehr beschränkt. Um eine solche Anpassbarkeit zu gewährleisten, müssten hier entsprechend unterschiedliche Bänder zur Verfügung stehen. Die Abstände zwischen den Intervertebralimplantaten ist vorgegeben.

Die EP 0 179 695 A offenbart ein Intervertebralimplantat, an dem Ösen angeformt sind, mit denen das Implantat an benachbarten Wirbeln festschraubbar ist. Auch hier ist die Anpassbarkeit an anatomische Gegebenheiten sehr beschränkt. Insbesondere ist es hier in der Regel nicht möglich, die Knochenschrauben an der geeigneten Stelle in den Wirbelkörper einzusetzen. Es können auch hier nur zwei Wirbel mit einem Intervertebralimplantat verbunden werden.

Die Stabilisierung von Intervertebralimplantaten mit herkömmlichen Platten erfordert somit wegen der grossen individuellen und krankheitsbedingten anatomischen Variabilität insbesondere der Halswirbelsäule ein grosses Sortiment verschieden langer Platten mit unterschiedlichen Lochabständen. Die Länge der Platten richtet sich dabei nach der Position der oberen und unteren Endlöcher. Wenn bei mehrsegmentalen Spondylodesen die Platten nur an die Endwirbel befestigt werden, besteht die Gefahr, dass die Platten an einem Endwirbel ausreissen. Es ist deshalb ratsame, die Platten auch an die zwischen den Endwirbeln liegenden Wirbelkörper mit Schrauben zu fixieren. Die bei herkömmlichen Platten dafür vorgesehenen Schraubenlöcher liegen aber nicht immer genau so, dass die betreffenden Schrauben gut in die zwischen den Endwirbeln liegenden Wirbelkörper eingesetzt werden können. Zudem kann bei verschiedenen Plattentypen nur eine Schraube implantiert werden.

Der Erfindung liegt die Aufgabe zugrunde, eine Vorrichtung der genannten Art zu schaffen, die optimaler an die jeweiligen anatomischen Gegebenheiten anpassbar ist.

Die Aufgabe ist bei einer gattungsgemässen Vorrichtung dadurch gelöst, dass das Intervertebralimplantat mit zwei im Abstand zueinander angeordneten Platten verbunden ist, wobei die beiden Platten jeweils an einem Ende mit dem Intervertebralimplantat ein fixierbares Gelenk bilden. Die beiden mit dem Intervertebralimplantat verbundenen Platten können unabhängig voneinander in mehreren Freiheitsgraden bewegt und in einer gewünschten Endstellung winkelstabil fixiert werden. Ein wesentlicher Vorteil der erfindungsgemässen Vorrichtung besteht darin, dass auch Mehretagenspondylodesen hergestellt werden können, wobei benachbarte Intervertebralimplantate direkt miteinander verbunden werden können. Dadurch ergibt sich eine besonders hohe Stabilität und damit Sicherheit. Die Intervertebralimplantate bilden somit mit den Platten eine besonders stabile Einheit. Es können auch unterschiedlich grosse Intervertebralimplantate kombiniert und mit unterschiedlichen Platten untereinander winkelstabil verbunden werden. Insbesondere ist eine Anpassung an unterschiedliche Krümmungen der Wirbelsäule und an unterschiedliche Abstände zwischen Intervertebralimplantaten und Wirbeln möglich.

Die Vorrichtung eignet sich insbesondere für die Spondylodese an der Halswirbelsäule. Hier empfiehlt es sich, die Spondylodese mit an die Vorderfläche der Halswirbelkörper fixierten Platten abzusichern. Es ist insbesondere ein Modularsystem möglich, das sowohl in der Herstellung als auch in der Praxis vorteilhaft ist. Die Anpassbarkeit ist mit einem überraschend kleinen Sortiment aus Einzelteilen möglich.

Nach einer Weiterbildung der Erfindung ist vorgesehen, dass wenigstens eine Platte Z-förmig oder L-förmig ausgebildet ist. Mit solchen Platten ist eine besonders hohe Anpassbarkeit und ein besonders stabiler Verbund möglich. Z-förmige Platten eignen sich insbesondere zum Verbinden benachbarter Intervertebralimplantate und damit zur Stabilisierung von drei Wirbeln. Solche Z-förmige Platten können in einem mittleren Bereich beispielsweise mit zwei Knochenschrauben verankert werden. L-förmige Platten ermöglichen ebenfalls eine Befestigung solcher Platten mit zwei Knochenschrauben.

Die Erfindung betrifft auch einen Bausatz zur Herstellung einer Vorrichtung gemäss Anspruch 1.

Ausführungsbeispiele der Erfindung werden nachfolgend anhand der Zeichnungen näher erläutert. Es zeigen:
- Fig. 1: schematisch eine räumliche Ansicht einer erfindungsgemässen Vorrichtung,
- Fig. 2: eine weitere Ansicht der erfindungsgemässen Vorrichtung gemäss Fig. 1,
- Fig. 3: einen Schnitt entlang der Linie III - III der Fig. 2,
- Fig. 4: eine weitere Ansicht der erfindungsgemässen Vorrichtung gemäss Fig. 1,
- Fig. 5: eine weitere Ansicht der erfindungsgemässen Vorrichtung gemäss Fig. 1,
- Fig. 6: eine räumliche Ansicht einer Variante der erfindungsgemässen Vorrichtung,
- Fig. 7: schematisch eine Ansicht eines Abschnittes einer Wirbelsäule mit einer erfindungsgemässen Vorrichtung, welche drei Wirbel miteinander verbindet, und
- Fig. 8: eine weitere schematische Ansicht des Abschnittes einer Wirbelsäule gemäss Fig. 7.

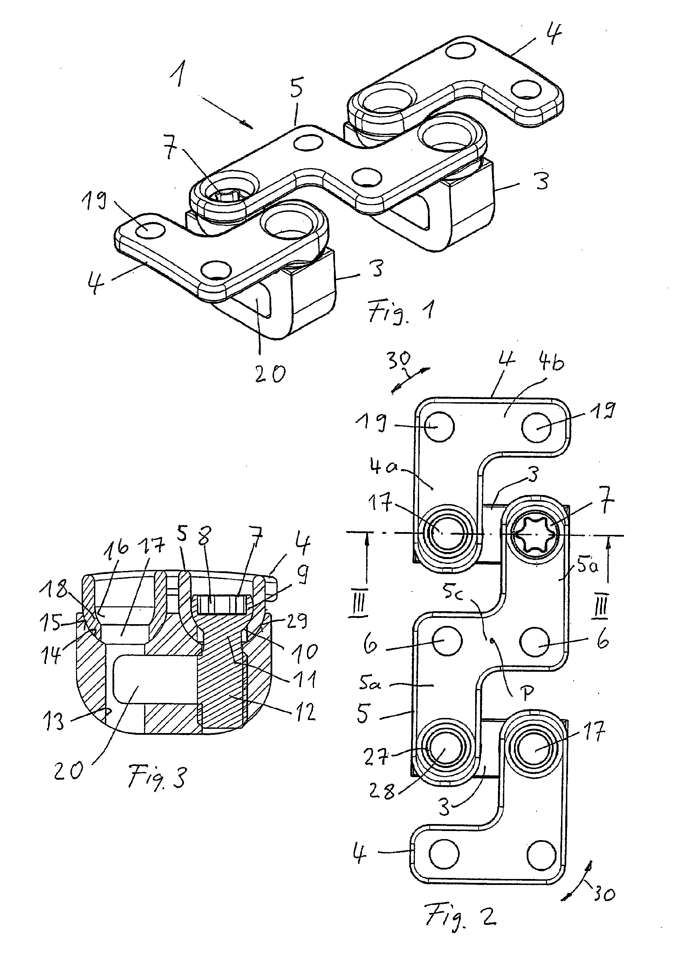

Die in den Fig. 1 - 4 gezeigte Vorrichtung 1 weist zwei Intervertebralimplantate 3 auf, die gemäss Fig. 7 jeweils in den Bandscheibenraum benachbarter Wirbel 21 und 22 bzw. 22 und 23 einer Wirbelsäule 24 eingesetzt sind. Die Wirbel 21 - 23 sind hier insbesondere Wirbel der Halswirbelsäule, wobei mit 26 die Vorderfläche der Halswirbelsäule bezeichnet ist. In der Fig. 7 liegen links vor der Vorderfläche hier nicht gezeigte Speiseröhre und grosse Blutgefässe.

Die beiden Intervertebralimplantate 3 bestehen aus Metall, insbesondere Titan oder einem geeigneten Kunststoff und sind interkorporelle und druckaufnehmende Körper. Sie weisen jeweils eine nach aussen offene Ausnehmung 20 auf, die zur Aufnahme von Knochen oder Knochenersatzmaterial dient. Die Ausnehmungen 20 werden vom Knochen des Patienten durchwachsen, so dass sich eine knöcherne Brücke zwischen den benachbarten Wirbeln 21 und 22 bzw. 22 und 23 bildet. Die Wirbel 21, 22 und 23 können damit fest miteinander verbunden werden.

Die beiden Intervertebralimplantate 3 sind mit einer Z-förmigen Platte 5 fest miteinander verbunden. Diese Platte 5 weist gemäss Fig. 2 zwei Arme 5a und 5b sowie einen quer zu diesen verlaufenden mittleren Bereich 5c auf. Die beiden Arme 5a und 5b sind vorzugsweise gleich ausgebildet, so dass die Platte 5 bezüglich eines mittleren Punktes P drehsymmetrisch ist. Die beiden Arme 5a weisen jeweils an einem freien Ende eine halbkugelförmige Vertiefung 27 auf, durch die mittig eine Durchgangsöffnung 28 hindurch geht. Diese halbkugelförmigen Vertiefungen 27 und die Durchgangsöffnungen nehmen jeweils eine Zylinderkopfschraube 7 auf, die mit einem Gewindeschaft 12 in eine Bohrung 13 eines Intervertebralimplantates 3 eingeschraubt ist. Die Zylinderkopfschrauben 7 weisen jeweils einen Kopf 9 mit einem Werkzeugangriff 8 und einer ebenfalls halbkugelförmigen Unterseite 10 auf. Zwischen dem Schaft 12 und dem Kopf 9 ist ein Hals 11 angeordnet, dessen Durchmesser kleiner ist als derjenige des Schaftes 12 und der Durchgangsöffnung 28. Die halbkugelförmige Unterseite 10 liegt an der halbkugelförmigen Vertiefung 27 an. Sind die beiden Schrauben 12 fest in das jeweilige Intervertebralimplantat 3 eingeschraubt, so ist die Platte 5 an ihren beiden freien Enden fest am jeweiligen Intervertebralimplantat 3 durch Klemmung fixiert. Die Klemmflächen werden durch eine halbkugelförmige Fläche 15 und eine ebenfalls halbkugelförmige Fläche 14 des entsprechenden Intervertebralimplantates 3 gebildet. Sind die beiden Zylinderkopfschrauben 7 nicht vollständig eingeschraubt, so bilden die beiden Verbindungen der Platte 5 zu den Intervertebralimplantaten 3 jeweils eine Art Kugelgelenk, die jeweils an beiden Befestigungspunkten Bewegungen in mehreren Freiheitsgraden ermöglichen.

Die Platte 5 und die beiden Intervertebralimplantate 3 können somit gegeneinander bewegt werden und in jeder gewünschten Position können diese Teile durch Festziehen der Schrauben 7 winkelstabil miteinander verbunden werden. Nach dem Festziehen der Zylinderkopfschrauben 7 bilden die beiden Intervertebralimplantate 3 und die Z-förmige Platte 5 eine stabile Einheit. Nötigenfalls können die Schrauben 7 jederzeit gelöst und damit wieder der bewegliche Zustand hergestellt werden.

An den beiden Intervertebralimplantaten 3 ist jeweils eine weitere Platte 4 angeordnet, die vorzugsweise L- bzw. winkelförmig ausgebildet sind. Diese Platten 4 weisen jeweils zwei Arme 4a und 4b auf, die gleich oder unterschiedlich sein können. Der Arm 4a weist an seiner Unterseite einen kugelförmigen Gelenkteil 18 mit einer kugelförmigen Innenfläche 16 und einer ebenfalls halbkugelförmigen Aussenfläche 15 auf. Der Gelenkteil 18 weist eine Durchgangsöffnung 17 auf. Der Gelenkteil 18 sitzt in einer halbkugelförmigen Vertiefung 14 des entsprechenden Intervertebralimplantates 3. Um die Platte 4 am entsprechenden Intervertebralimplantat 3 zu befestigen, wird gleich wie zur Befestigung der Z-förmigen Platte 5 in den Gelenkteil 18 von oben eine Zylinderkopfschraube 7 eingesetzt und in die Bohrung 13 des entsprechenden Intervertebralimplantates 3 eingeschraubt. In den Fig. 1 - 3 sind diese Schrauben 7 aus zeichnerischen Gründen nicht eingezeichnet. Vor dem Festziehen der Schraube 7 kann die entsprechende Platte 4 aufgrund der kugelgelenkartigen Verbindung bewegt und an die anatomischen Gegebenheiten angepasst werden. Ist die optimale Position erreicht, so werden die entsprechenden Schrauben 7 angezogen und damit die Platten 4 winkelstabil bezüglich des entsprechenden Intervertebralimplantates 3 fixiert.

Die beiden Platten 4 weisen im Arm 4b jeweils zwei im Abstand zueinander angeordnete Durchgangsöffnung 19 auf, die jeweils zur Aufnahme einer Knochenschraube 25 dienen, wie dies in Fig. 7 schematisch dargestellt ist. Solche Knochenschrauben 25 sind an sich bekannt. Die Verbindung kann mit einer Schraube gemäss der WO 01/30251 winkelstabil oder alternativ auch beweglich sein. Mit diesen Schrauben 25 werden die beiden Platten 4 im entsprechenden Wirbelkörper 21 bzw. 23 befestigt. Diese Knochenschrauben 25 sollten möglichst in der Mitte des entsprechenden Wirbelkörpers 21 bzw. 23 liegen. Andernfalls könnte ihr Halt beeinträchtigt werden. Diese Schrauben sollten ferner nicht in eine gesunde Bandscheibe eindringen und zudem sollten die Arme 4b nicht eine gesunde Bandscheibe berühren. Dies würde zu Zerstörungen bzw. Degenerationen der betreffenden Bandscheiben führen. Aufgrund der Einstellbarkeit der Platten 4 ist es nun möglich, diese so auszurichten, dass die Schrauben wie oben erläutert in geeigneten Positionen in die Wirbelkörper 21 bzw. 23 eingeschraubt werden können. Ebenfalls kann damit vermieden werden, dass die Arme 4b eine Bandscheibe berühren.
Die genannte Einstellbarkeit der Platten 4 lassen sich diese an die unterschiedlichen Krümmungen und Formen der Vorderfläche 26 der Halswirbelsäule anpassen. Dies ist auch dann möglich, wenn Intervertebralimplantate 3 wie gewünscht nicht an der Vorderseite 26 vorstehen. Die Intervertebralimplantate 3 dürfen an der Vorderfläche 26 nicht vorstehen, da sie dadurch Verletzungen der vor der Halswirbelsäule 24 angeordneter Gebilde und insbesondere der Speiseröhre und grosser Blutgefässe verursachen könnten.

Die beiden Platten 4 verbinden somit jeweils ein Intervertebralimplantat 3 mit einem Wirbelkörper 21 und 22 bzw. 22 und 23. Die Platte 5 verbindet die beiden Intervertebralimplantate 3 winkelstabil miteinander. Zudem weist die Platte 5 im mittleren und quer verlaufen Bereich 5c zwei Durchgangsöffnungen 6 auf, welche jeweils eine Knochenschraube 25 aufnehmen, die in den mittleren Wirbelkörper 22 eingeschraubt sind und mit denen dadurch die Platte 5 an diesem mittleren Wirbel 22 verankert wird. Auch hier ist eine winkelstabile oder bewegliche Verbindung zwischen Platte 4 und Knochenschraube 25 möglich.

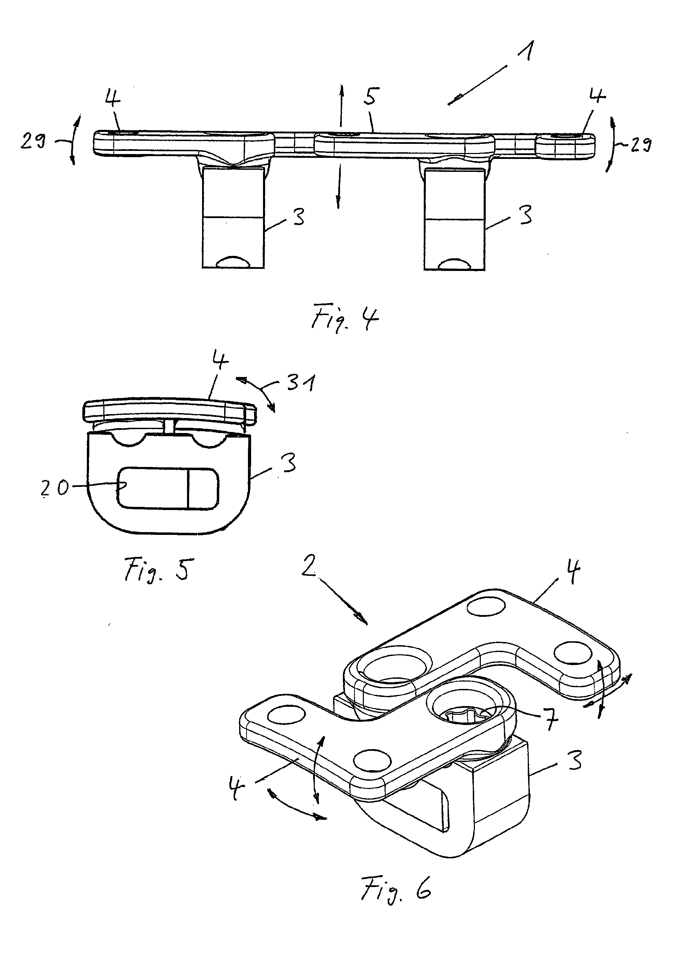

Die beiden Platten 4 und die Platte 5 können gemäss den Fig. 4 und 5 in einer Ebene liegen. Aufgrund der genannten Gelenkverbindungen ist eine solche ebene Anordnung jedoch nicht zwingend. So können die beiden Platten 4 gemäss den Doppelpfeilen 29 der Fig. 4 unabhängig voneinander in einen vergleichsweise grossen Bereich nach oben und nach unten verschwenkt werden. Diese beiden Platten 4 können auch unabhängig voneinander gemäss den Doppelpfeilen 30 der Fig. 2 verschwenkt werden. Aufgrund der kugelgelenkartigen Verbindungen sind hier aber auch noch andere Bewegungen bzw. Freiheitsgrade möglich. So steht insbesondere die in Fig. 5 mit dem Doppelpfeil 31 angedeutete Schwenkbewegung zur Verfügung. Jede mögliche Position ist durch Festziehen der entsprechenden Zylinderkopfschraube 7 fixierbar. Damit ist wie oben erläutert eine optimale Anpassung an die jeweiligen anatomischen Gegebenheiten möglich.

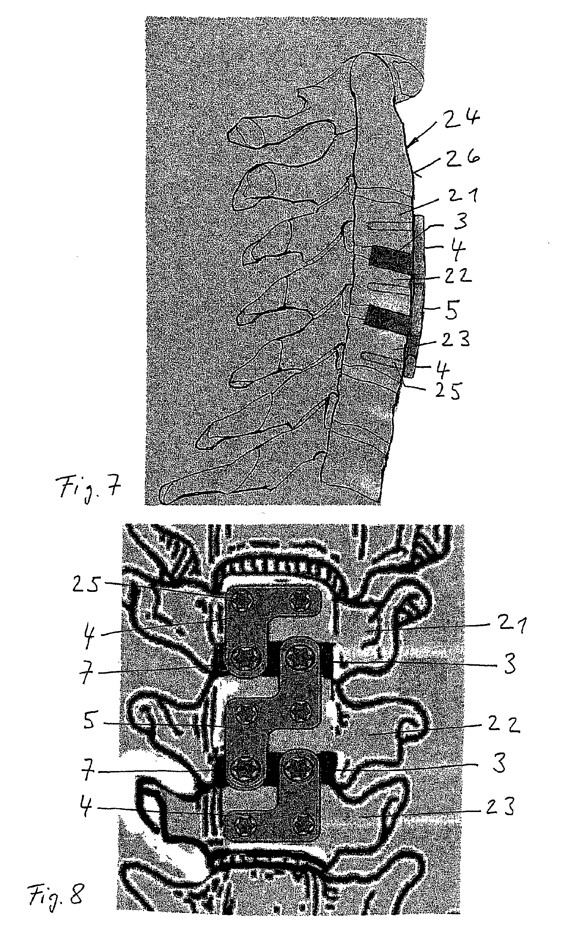

Die Fig. 6 zeigt eine erfindungsgemässe Vorrichtung 2, die lediglich ein Intervertebralimplantat 3 sowie zwei L- bzw. winkelförmige Platten 4 aufweist. Die Platten 4 sind wie bereits oben erläutert jeweils mit einer Zylinderkopfschraube 7 am Intervertebralimplantat 3 befestigt. Die Verbindung ist wie oben erläutert kugelgelenkartig. Die beiden Platten 4 können ebenfalls wie oben erläutert vor dem Festziehen der beiden Schrauben 7 bewegt werden. In der Fig. 6 ist lediglich eine der beiden Zylinderkopfschrauben 7 gezeigt. Die beiden Platten 4 sind in der gezeigten Ausführung gleich ausgebildet. Die Platten 4 können jedoch auch unterschiedlich sein, beispielsweise unterschiedlich lang sein. Sie müssen nicht zwingend L- bzw. winkelförmig sein, sondern können auch gerade längliche Platten sein. Dies gilt auch für die Platten 4 der Vorrichtung 1. Die Vorrichtung 2 dient zum Verbinden von zwei benachbarten Wirbeln 21 und 22 bzw. 22 und 23. Selbstverständlich können an einer Wirbelsäule 24 mehrere solche Vorrichtungen 2 angebracht werden. Die entsprechenden Intervertebralimplantate 3 sind dann jedoch nur über zwischenliegende Wirbel miteinander verbunden. Eine direkte Verbindung wie bei der Vorrichtung 1 ist damit hier nicht vorhanden.

Die Platten 4 und 5 sowie die Intervertebralimplantate 3 sind vorzugsweise aus einem geeigneten Kunststoff, beispielsweise aus einem faserverstärkten Kunststoff hergestellt. Solche Kunststoffe sind an sich bekannt und haben den wesentlichen Vorteil, dass sie für Röntgenstrahlen durchlässig sind. Mit solchen Materialien können auch sehr stabile Platten 4 und 5 hergestellt werden, die zudem sehr biegesteif sind. Damit ist es möglich, Bewegungen innerhalb der Spondylodese und damit das Risiko einer mitunter folgenschweren sekundären Implantatdislokation zu mindern. Grundsätzlich können die Platten 4 und 5 aber auch aus einem anderen Werkstoff, beispielsweise auch aus Titan hergestellt werden.

Möglich ist auch eine hier nicht gezeigte Vorrichtung mit mehr als zwei Intervertebralimplantaten 3, die mit einer entsprechenden Anzahl von Z-förmigen Platten 5 direkt miteinander verbunden sind. Beispielsweise kann eine solche Vorrichtung drei Intervertebralimplantate 3 aufweisen, die mit zwei Z-förmigen Platten 5 miteinander verbunden sind. An den beiden äusseren Intervertebralimplantaten ist dann jeweils noch eine L-förmige Platte 4 befestigt.

Nachfolgend wird ein Verfahren zur Versteifung eines Abschnittes einer Wirbelsäule mit der erfindungsgemässen Vorrichtung näher erläutert.

Nach einer entsprechenden Freilegung des zu versteifenden Abschnittes der Wirbelsäule wird die befallene Bandscheibe ausgeräumt. Mit einem geeigneten Instrument wird der ausgeräumte Bandscheibenraum erweitert bzw. vergrössert. Nun wird ein passendes Intervertebralimplantat 3 ausgewählt und in den ausgeräumten Bandscheibenraum eingesetzt, wo es zwischen den benachbarten Wirbeln verklemmt. Gegebenenfalls wird eine weitere Bandscheide ausgeräumt und entsprechend ein weiteres Intervertebralimplantat 3 eingesetzt.

Nun werden Abstände zwischen dem Intervertebralimplantat 3 bzw. den Intervertebralimplantaten 3 und geeigneten Positionen für die Knochenschrauben ermittelt. Bei zwei Intervertebralimplantaten 3 wird auch der Abstand zwischen diesen gemessen. Entsprechend diesen Abständen werden passende Platten 4 und gegebenenfalls 5 gewählt und diese lose am Intervertebralimplantat 3 bzw. an den Intervertebralimplantaten fixiert. Die Platten 4 werden so gewählt, dass sie benachbarte Bandscheiben nicht berühren, wie dies in den Figuren 7 und 8 ersichtlich ist. Die lose fixierten Platten 4 und 5 werden an die Wirbel angelegt und mit Knochenschrauben 25 fixiert. Nun werden die Platten 4 und gegebenenfalls 5 durch Festziehen der Schrauben 7 fest mit dem Intervertebralimplantat 3 bzw. Intervertebralimplantaten verbunden.

Die Figuren 7 und 8 zeigen drei Wirbel 21 bis 23, die mit einer erfindungsgemässen Vorrichtung miteinander verbunden sind. Die Platte 5 verbindet wie ersichtlich zwei Intervertebralimplantate 3 miteinander und ist zudem mit Knochenschrauben 25 mit dem Wirbel 22 verbunden. Werden lediglich zwei Wirbel miteinander verbunden, wird die Vorrichtung 2 gemäss Figur 6 verwendet, bei der eine Z-förmige Platte 5 nicht vorgesehen ist.

## Patentansprüche

1. Vorrichtung für die Spondylodese und insbesondere für die vordere interkorporelle Spondylodese an der Halswirbelsäule, mit wenigstens einem Intervertebralimplantat (3) und mit wenigstens einer Platte (4,5), die mit dem Intervertebralimplantat (3) und einem benachbarten Wirbel (21-23) zu verbinden ist, **dadurch gekennzeichnet, dass** das Intervertebralimplantat (3) mit wenigestens zwei im Abstand zueinander angeordneten Platten (4,5) verbunden ist, wobei die beiden Platten (4,5) jeweils an einem Ende mit dem Intervertebralimplantat (3) ein fixierbares Gelenk bilden.

2. Vorrichtung nach Anspruch 1, **dadurch gekennzeichnet, dass** wenigstens eine der Platten (5,4) Z-förmig oder L-förmig ausgebildet ist.

3. Vorrichtung nach Anspruch 1 oder 2, **dadurch gekennzeichnet, dass** wenigstens eine Platte (5) in einem mittleren und quer zur Längsachse der Wirbelsäule (24) verlaufenden Bereich (5c) wenigstens einen Durchgang (6) für eine Knochenschraube (25) aufweist, derart, dass diese Platte (5) mit einem Wirbel (22) verbindbar ist.

4. Vorrichtung nach Anspruch 3, **dadurch gekennzeichnet, dass** der genannte Bereich (5c) zwei im Abstand zueinander angeordnete Durchgänge (6) für jeweils eine Knochenschraube (25) aufweist.

5. Vorrichtung nach einem der Ansprüche 1 - 4, **dadurch gekennzeichnet, dass** wenigstens eine Platte (4,5) an wenigstens einem Ende einen halbkugelförmigen und vorstehenden Gelenkteil (18) aufweist, der einen Durchgang (17) für eine Befestigungsschraube (7) aufweist und der in eine halbkugelförmige Vertiefung (14) eines Intervertebralimplantates (3) eingreift, derart, dass ein Kugelgelenk gebildet wird.

6. Vorrichtung nach Anspruch 5, **dadurch gekennzeichnet, dass** wenigstens zwei Platten (4,5) jeweils über ein Kugelgelenk mit einem Intervertebralimplantat (3) verbunden sind.

7. Vorrichtung nach einem der Ansprüche 1 - 6, **dadurch gekennzeichnet, dass** wenigstens zwei L-förmige Platten (4) mit einem Intervertebralimplantat (3) verbunden sind.

8. Vorrichtung nach Anspruch 7, **dadurch gekennzeichnet, dass** die beiden Platten (4) jeweils in einem quer zur Längsrichtung der Wirbelsäule (24) verlaufenden Arm (4b) wenigstens zwei im Abstand zueinander angeordnete Durchgänge (19) zur Aufnahme jeweils einer Knochenschraube (25) aufweisen.

9. Vorrichtung nach einem der Ansprüche 1 - 8, **dadurch gekennzeichnet, dass** zwei Intervertebralimplantate (3) mit einer Z-förmigen Platte (5) miteinander verbunden sind.

10. Vorrichtung nach einem der Ansprüche 1 - 9, **dadurch gekennzeichnet, dass** zwei Intervertebralimplantate (3) vorgesehen sind, die mit einer Z-förmigen Platte (5) miteinander verbunden sind und an denen jeweils eine L-förmige Platte (4) befestigt ist, wobei sämtliche Verbindungen zwischen den Platten (4,5) und den Intervertebralimplantaten (3) als Kugelgelenke ausgebildet sind.

11. Vorrichtung nach einem der Ansprüche 1 - 10, **dadurch gekennzeichnet, dass** wenigstens eine Platte (4,5) und/oder ein Intervertebralimplantat (3) aus einem röntgendurchlässigen Werkstoff hergestellt ist bzw. sind.

12. Vorrichtung nach einem der Ansprüche 1 - 11, **dadurch gekennzeichnet, dass** sie für eine Spondylodese der Halswirbelsäule vorgesehen ist.

13. Bausatz zur Herstellung einer Vorrichtung nach einem der Ansprüche 1 - 12, mit wenigstens einem Intervertebralimplantat (3) und wenigstens einer Platte (4,5) zum Verbinden des Intervertebralimplantates (3) mit wenigstens einem Wirbel (21-23), **dadurch gekennzeichnet, dass** wenigstens eine Platte (4) L-förmig und wenigstens eine Platte (5) Z-förmig ausgebildet ist und dass wenigstens ein Intervertebralimplantat (3) im Abstand zueinander zwei Bohrungen (13) zur Aufnahme jeweils einer Befestigungsschraube (7) aufweist.

14. Bausatz nach Anspruch 13, **dadurch gekennzeichnet, dass** das Intervertebralimplantat (3) und die Platten (4,5) jeweils mindestens ein Gelenkteil (18) zum Bilden eines Kugelgelenkes aufweisen.

15. Bausatz nach Anspruch 13 oder 14, **dadurch gekennzeichnet, dass** das Intervertebralimplantat (3) und die Platten (4,5) aus einem röntgendurchlässigen Werkstoff hergestellt sind.

16. Bausatz nach einem der Ansprüche 13 - 15, **gekennzeichnet durch** mehrere Knochenschrauben (25) und mehrere Befestigungsschrauben (7).

17. Bausatz nach Anspruch 16, **dadurch gekennzeichnet, dass** die Befestigungsschrauben Zylinderkopfschrauben sind, die einen Schraubenkopf (8) aufweisen, der an seiner Unterseite im Wesentlichen halbkugelförmig ausgebildet ist.
